(19) Europäisches Patentamt
European Patent Office
Office européen des brevets

(11) Veröffentlichungsnummer: **0 073 997**
**A1**

(12) # EUROPÄISCHE PATENTANMELDUNG

(21) Anmeldenummer: 82107679.1

(22) Anmeldetag: 23.08.82

(51) Int. Cl.³: C 07 D 211/84
C 07 D 221/10, C 07 D 211/90
A 61 K 31/44, C 07 D 215/38

(30) Priorität: 03.09.81 DE 3134945

(43) Veröffentlichungstag der Anmeldung: 16.03.83 Patentblatt 83/11

(84) Benannte Vertragsstaaten: AT BE CH DE FR GB IT LI LU NL SE

(71) Anmelder: BAYER AG
Zentralbereich Patente, Marken und Lizenzen
D-5090 Leverkusen 1, Bayerwerk(DE)

(72) Erfinder: Rosentreter, Ulrich, Dr.
Kondorweg 23
D-5600 Wuppertal 1(DE)

(72) Erfinder: Puls, Walter, Dr.
In den Birken 75
D-5600 Wuppertal 1(DE)

(72) Erfinder: Bischoff, Hilmar, Dr.
Wilkhausstrasse 107
D-5600 Wuppertal 2(DE)

(54) **Substituierte 2-Amino-3,4-dihydropyridinderivate, Verfahren zu ihrer Herstellung und ihre Verwendung in Arzneimitteln.**

(57) Die vorliegende Erfindung betrifft neue substituierte 2-Amino-3,4-dihydropyridinderivate der allgemeinen Formel I

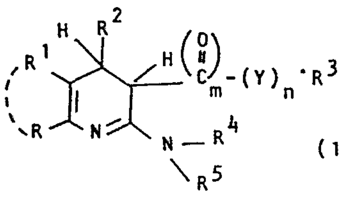

(1)

in welcher R, $R^1$, $R^2$, $R^3$, $R^4$, $R^5$, Y, m und n die in der Beschreibung angegebene Bedeutung haben, ein Verfahren zu ihrer Herstellung sowie ihre Verwendung als Arzneimittel, insbesondere ihre Verwendung bei der Bekämpfung von Stoffwechselstörungen.

EP 0 073 997 A1

0073997

BAYER AKTIENGESELLSCHAFT 5090 Leverkusen, Bayerwerk

Zentralbereich
Patente, Marken und Lizenzen KS-by-c
Ia (Pha)

# Substituierte 2-Amino-3,4-dihydropyridinderivate, Verfahren zu ihrer Herstellung und ihre Verwendung in Arzneimitteln

Die vorliegende Erfindung betrifft neue substituierte 2-Amino-3,4-dihydropyridinderivate, ein Verfahren zu ihrer Herstellung sowie ihre Verwendung als Arzneimittel, insbesondere ihre Verwendung bei der Bekämpfung von Stoffwechselstörungen.

Es ist bereits bekannt, daß 3,5-Bisalkoxycarbonyl-2-alkylaminodihydropyridine kreislaufbeeinflussende Wirkungen, insbesondere blutdrucksenkende Wirkung besitzen (vgl. DT-OS 2 239 815 und Brit.Pat. 1 384 ,219). In der DT-OS 2 949 701 wird für einige Dihydropyridinderivate eine lipidabsorptionshemmende Wirkung beschrieben. Die neuen erfindungsgemäßen Verbindungen zeigen in unerwarteter Weise eine vorteilhafte Wirkung auf bestimmte Stoffwechselerkrankungen.

Die vorliegende Anmeldung betrifft neue substituierte 2-Amino-3,4-dihydropyridinderivate der allgemeinen Formel (I)

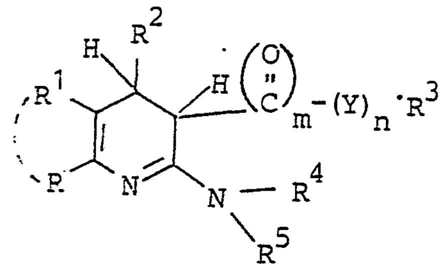 (I)

in welcher

R für Wasserstoff, Alkyl, gegebenenfalls substituiert durch Hydroxy, Halogen, Alkoxy, Aryl oder die Gruppe

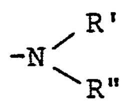 steht,

wobei R' und R" gleich oder verschieden sind und jeweils Wasserstoff, Alkyl oder Aralkyl bedeuten, oder für gegebenenfalls substituiertes Aralkyl steht,

oder für Aryl steht, welches gegebenenfalls durch 1 oder 2 gleiche oder verschiedene Substituenten aus der Gruppe Halogen, Alkyl, Alkoxy, Alkylmercapto oder Trifluormethyl substituiert ist, oder

R und $R^1$ gemeinsam gegebenenfalls einen carbocyclischen Ring bilden, der gegebenenfalls substituiert ist durch Hydroxy, Halogen, Nitro, Alkyl, Alkoxy oder die

Gruppe 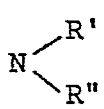, wobei R' und R" die oben angeführten Bedeutungen haben, und wobei an den carbocyclischen Ring ein Arylring ankondensiert sein kann, der gegebenenfalls substituiert ist durch 1 oder 2 gleiche oder verschiedene Substituenten aus der Gruppe Halogen, Alkyl, Alkoxy, Alkylmercapto oder Trifluormethyl,

und wobei der Arylring gegebenenfalls durch Sauerstoff, Schwefel oder die $NR^6$-Gruppe unterbrochen ist, wobei $R^6$ die für $R^1$ und $R^2$ angegebene Bedeutung besitzt,

$R^1$ und $R^2$ gleich oder verschieden sind und jeweils für Wasserstoff oder Alkyl, gegebenenfalls substituiert durch Aryl, Heteroaryl, Hydroxyl, Halogen, Alkoxy, Carboxy, Cyano, Carbalkoxy oder die Gruppe 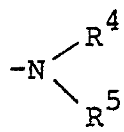 mit den unten angeführten Bedeutungen für $R^4$ und $R^5$ stehen,

oder

für einen Arylrest oder für einen Heteroarylrest aus der Gruppe Thienyl, Furyl, Pyrryl, Pyrazolyl, Imidazolyl, Oxazolyl, Isooxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl stehen, wobei der Arylrest sowie die Heteroarylreste gegebenenfalls 1, 2 oder 3 gleiche oder verschiedene Substituenten aus der Gruppe Phenyl, Alkyl, Alkenyl, Alkinyl, Alkoxy, Alkenoxy, Alkinoxy, Alkylen, Dioxyalkylen, Halogen, Acyloxy, Trifluormethylthio, Trifluormethyl, Trifluormethoxy, Hydroxy,

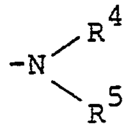 ,

mit den unten angegebenen Bedeutungen für $R^4$ und $R^5$, Nitro, Cyano, Azido, Carboxy, Carbalkoxy, Carbonamido, Sulfonamido oder $SO_{m'}$-Alkyl (m' = 0, 1 oder 2) enthalten, und wobei die Alkyl- und Alkoxysubstituenten ihrerseits gegebenenfalls substituiert sind durch Hydroxy, Alkoxy, Carboxy, Carbalkoxy, Halogen, die

Gruppe 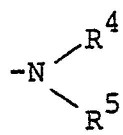 ,

mit den unten angegebenen Bedeutungen für $R^4$ und $R^5$, Aroyloxy oder Alkanoyloxy, wobei der Aroyl- und Alkanoylrest durch Hydroxy, Alkoxy, Halogen, Carboxy oder Carbalkoxy substituiert sein kann,

m und n gleich oder verschieden sind und jeweils 0 oder 1 bedeuten,

Y für Sauerstoff, die Gruppe $SO_m$-$R^6$, (m' = 0, 1, 2) oder die Gruppe N-$R^6$, wobei $R^6$ einen Alkyl- oder Arylrest bedeutet, steht, und

$R^3$ für Alkyl, gegebenenfalls substituiert durch Hydroxy, Alkoxy, Halogen, die Gruppe

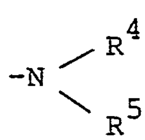 , mit den unten angeführten Bedeutungen

für $R^4$ und $R^5$, Carboxy, Carbalkoxy, Cyano, Alkenyl oder Aryl steht,
oder
für Aryl, welches gegebenenfalls substituiert ist durch 1 oder 2 gleiche oder verschiedene Substituenten der Gruppe Hydroxy, Alkoxy, Halogen, Alkyl, Alkenyl, Aryl, Alkylmercapto, Alkylamino, Carboxy, Carbalkoxy, Cyano, Trifluormethyl, Trifluormethoxy, Trifluormethylthio, steht
oder

$R^3$ auch für Wasserstoff steht, wenn

a) R und $R^1$ gemeinsam einen Ring bilden oder
b) $R^1$ und $R^2$ für Wasserstoff oder gegebenenfalls substituiertes Alkyl stehen, oder
c) $R^1$ und $R^2$ für $R^{1'}$ und $R^{2'}$ stehen, wobei mindestens einer dieser Substituenten für Aryl steht, welches 1, 2 oder 3 gleiche oder verschiedene Substituenten aus der Gruppe Acyloxy, Arylmercapto, Hydroxyalkyl, Hydroxyalkoxy, Hydroxyalkylmercapto und $NR^{4'}R^{5'}$ trägt,
wobei
die Hydroxylgruppen der Alkyl- und Alkoxysubstituenten gegebenenfalls ihrerseits substituiert sind durch Aroyl oder Alkanoyl, die ihrerseits wieder eine Carboxy- oder Carbalkoxygruppe als Substituenten tragen können, und
wobei
$R^{4'}$ und $R^{5'}$ mit dem Stickstoffatom einen 4- bis 7-gliedrigen Ring bilden, der gegebenenfalls durch Sauerstoff, Schwefel,

die NH-Gruppe oder die $NR^6$-Gruppe unterbrochen ist, wobei $R^6$ die oben angegebene Bedeutung besitzt, und

$R^4$ und $R^5$ gleich oder verschieden sind und jeweils für Wasserstoff oder für einen Alkyl-, Aralkyl-, Alkenyl- oder Alkinylrest stehen, wobei die Alkyl- und Alkenylreste geradkettig, verzweigt oder cyclisch sind und gegebenenfalls substituiert sind durch die Gruppe $COR^5$, wobei $R^5$ Alkyl, Aralkyl oder Aryl bedeutet,

oder

$R^4$ und $R^5$ gemeinsam mit dem Stickstoffatom einen 4- bis 7-gliedrigen Ring bilden, der gegebenenfalls durch Sauerstoff, Schwefel, die NH-Gruppe oder die $NR^6$-Gruppe unterbrochen ist, wobei $R^6$ die oben angegebene Bedeutung besitzt,

sowie ihre pharmazeutisch unbedenklichen Salze.

Es wurde gefunden, daß man die Verbindungen der allgemeinen Formel (I) erhält, wenn man α,ß-ungesättigte Oxoverbindungen der allgemeinen Formel (II)

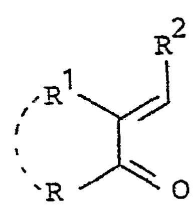

(II)

in welcher

R, $R^1$ und $R^2$ die oben angegebene Bede ung haben,

mit einem 3,3-Diamino-acrylsäureester der allgemeinen Formel (IIIa)

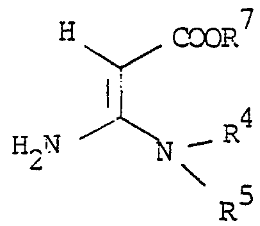

(IIIa)

bzw. ihrer tautomeren Iminform der allgemeinen Formel (IIIb)

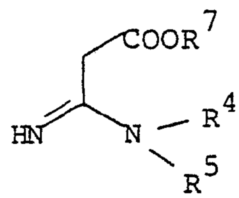

(IIIb)

in welcher

$R^4$ und $R^5$ die oben angegebene Bedeutung haben und

$R^7$ für einen gegebenenfalls substituierten Alkyl- oder Aralkylrest steht,

in Gegenwart von inerten organischen Lösungsmitteln und gegebenenfalls in Gegenwart von sauren oder basischen Katalysatoren bei Temperaturen zwischen 20 und 150°C umsetzt, und die dann erhaltenen Verbindungen der Formel (IV)

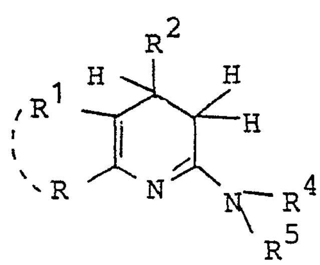

(IV)

in welcher

$R, R^1, R^2, R^4$ und $R^5$ die oben angegebene Bedeutung haben, und für die Herstellung von erfindungsgemäßen Verbindungen, die in

3-Position substituiert sind,

gegebenenfalls mit Verbindungen der allgemeinen Formel (V)

$$X-\left(\overset{O}{\overset{\|}{C}}\right)_m-(Y)_n-R^3 \qquad (V)$$

in welcher

m,n,Y und $R^3$ die oben angegebene Bedeutung haben, und

Y für Halogen, Alkoxy, Aryloxy, Alkylmercapto, Arylmercapto, Toxyl, Mexyl oder die Gruppe $(CO)_m-(Y)_n-R^3$ steht,

in Gegenwart einer starken Base und in Gegenwart eines inerten, aprotischen Lösungsmittels bei Temperaturen zwischen -100°C und 80°C umsetzt.

Verwendet man 1-(4-Chlorbenzyliden)-2-tetralon und 3-Amino-3-$N^4$-phenylpiperazinoacrylsäureethylester und Benzylbromid als Ausgangsverbindungen, so kann der Reaktionsablauf durch folgendes Formelschema wiedergegeben werden:

a)

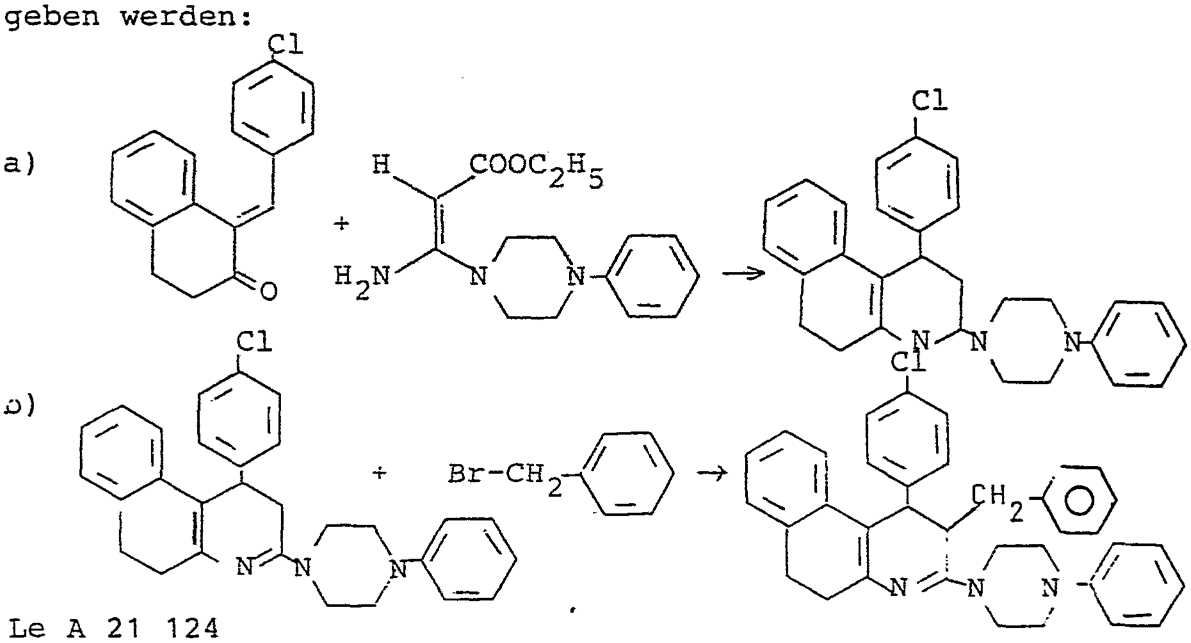

b)

Die erfindungsgemäß einsetzbaren α,ß-ungesättigten Oxoverbindungen der allgemeinen Formel (III) sind bekannt oder können nach bekannten Methoden hergestellt werden (vgl. J. Am. Chem. Soc. 81, 113 (1959)).

Die als Ausgangsverbindungen verwendbaren 3,3-Diaminoacrylsäureester der allgemeinen Formel (IIIa) bzw. ihre tautomeren Iminformen (IIIb) sind ebenfalls bekannt oder können nach bekannten Methoden hergestellt werden (vgl. DT-OS 2 239 815 und H. Meyer, F. Bossert, H. Horstmann, Liebigs Ann. Chem. 1977, 1895).

Die erfindungsgemäß einsetzbaren Verbindungen der allgemeinen Formel (V) sind bekannt oder können nach bekannten Methoden hergestellt werden.

Die aus den Ausgangsverbindungen der allgemeinen Formel (II) und (III) gebildeten Zwischenprodukt der allgemeinen Formel (IV) sind teilweise bekannt oder können nach bekannten Methoden hergestellt werden (vgl. DT-OS 2 949 701).

Bei der Durchführung des erfindungsgemäßen ersten Verfahrensschrittes kommen als Verdünnungsmittel alle inerten organischen Lösungsmittel in Frage. Hierzu gehören vorzugsweise niedere Alkohole wie Methanol, Ethanol, Propanol; Ether wie Dioxan, Diethylether; Ketone wie Aceton; Eisessig, Pyridin, Dimethylformamid, Dimethylsulfoxid oder Acetonitril.

Die Reaktionstemperaturen können in einem größeren Bereich variiert werden. Im allgemeinen arbeitet man zwischen 20 und 150°C, vorzugsweise bei 30 bis 120°C, insbesondere bei der Siedetemperatur des Lösungsmittels.

Als saure Katalysatoren seien vorzugsweise genannt: Mineralsäuren, wie Schwefelsäure oder Phosphorsäure, Sulfonsäure wie Toluolsulfonsäure und als basische Katalysatoren seien vorzugsweise genannt Alkali- und Erdalkalihydroxide und Alkoholate, insbesondere Alkalimetallalkoholate.

Die Umsetzung kann bei Normaldruck, aber auch bei erhöhtem Druck, durchgeführt werden. Im allgemeinen arbeitet man bei Normaldruck.

Bei der Durchführung des Verfahrens werden die an der Reaktion beteiligten Stoffe vorzugsweise jeweils in molaren Mengen eingesetzt.

Bei der Durchführung des erfindungsgemäßen zweiten Verfahrensschrittes kommen als Verdünnungsmittel alle inerten, aprotischen, organischen Lösungsmittel in Frage. Hierzu gehören vorzugsweise niedere Ether wie Tetrahydrofuran, Dioxan, Diethylether, 1,2-Dimethoxyethan, Kohlenwasserstoffe, wie Hexan, Cyclohexan, Benzol, Toluol; Dimethylformamid, Dimethylsulfoxid oder Hexamethylphosphorsäuretriamid.

Die Reaktionstemperaturen können in einem größeren Bereich variiert werden. Im allgemeinen arbeitet man zwischen -100°C und 80°C, vorzugsweise zwischen -80°C und 20°C.

Als Basen seien vorzugsweise genannt Alkali- und Erdalkaliamide, insbesondere Alkalimetallamide, Alkali- und Erdalkalihydride, insbesondere Alkalimetallhydride, Alkali- und Erdalkalialkylverbindungen, insbesondere Alkalimetallalkyle, Alkali- und Erdalkaliarylverbindungen, insbesondere Alkalimetallaryle.

Die Umsetzung kann bei Normaldruck, aber auch bei erhöhtem Druck durchgeführt werden. Im allgemeinen arbeitet man bei Normaldruck. Bei der Durchführung des Verfahrens werden die an der Reaktion beteiligten Stoffe in gleichen molaren Mengen oder aber 1 oder 2 Komponenten im Überschuß eingesetzt.

Die Umsetzung wird im allgemeinen unter einer Inertgasatmosphäre durchgeführt. Als Inertgas kommen Stickstoff oder Argon in Frage.

Wenn nicht ausdrücklich anders angegeben steht Alkyl in der vorliegenden Anmeldung für geradkettiges, verzweigtes oder cyclisches Alkyl mit bis zu 10 Kohlenstoffatomen, insbesondere für geradkettiges oder verzweigtes Alkyl mit bis zu 6 Kohlenstoffatomen, Aryl bedeutet vorzugsweise Phenyl oder Naphthyl und Aralkyl steht vorzugsweise für Benzyl, Phenethyl oder Phenylpropyl. Alkoxy steht für geradkettiges oder verzweigtes Alkoxy mit 1 bis 6 Kohlenstoffatomen und Halogen bedeutet vorzugsweise Fluor, Chlor oder Brom.

Von besonderem Interesse sind Verbindungen der allgemeinen Formel (I) in welcher

R für Wasserstoff, geradkettiges oder verzweigtes Alkyl mit bis zu 6 Kohlenstoffatomen steht, welches gegebenenfalls 1- oder 2-fach substituiert ist durch Phenyl,Hydroxy, Alkoxy mit 1 bis 4 Kohlenstoffatomen, Amino, Monoalkylamino, Dialkylamino oder Benzylalkylamino, wobei die genannten Alkylgruppen 1 bis 4 Kohlenstoffatome enthalten oder für einen Phenyl- oder Benzylrest steht, wobei der Phenylring gegebenenfalls 1- oder 2-fach substituiert ist durch gleiche oder verschiedene Substituenten aus der Gruppe Fluor, Chlor, Brom, Alkyl, Alkoxy, Alkylmercapto mit jeweils 1 bis 4 Kohlenstoffatomen oder durch Trifluormethyl und

R und $R^1$ gemeinsam gegebenenfalls einen carbocyclischen Ring mit 5 bis 7 Ringgliedern bilden, an den ein Benzol- oder Naphthalinring ankondensiert ist, wobei der Benzol- bzw. Naphthalinring gegebenenfalls 1- oder 2-fach substituiert ist durch gleiche oder verschiedene Substituenten aus der Gruppe Fluor, Chlor, Brom, Trifluormethyl, Alkyl oder Alkoxy mit jeweils 1 bis 4 Kohlenstoffatomen, und der gegebenenfalls durch Sauerstoff, Schwefel, die Gruppe NH oder ein alkylsubstituiertes Stickstoffatom mit 1 bis 4 Kohlenstoffatomen unterbrochen ist,

$R^1$ und $R^2$ gleich oder verschieden sind und jeweils für Wasserstoff, geradkettiges und verzweigtes Alkyl, welcher gegebenenfalls 1 oder 2 gleiche oder verschiedene Substituenten aus der Gruppe Phenyl, Hydroxy, Amino, Carboxy, Halogen, Alkylamino, Dialkylamino mit jeweils 1 bis 4 C-Atomen trägt, oder für Phenyl, Naphthyl, Furyl oder Pyridyl stehen, wobei diese aromatischen Ringe gegebenenfalls 1 oder 2 gleiche oder verschiedene Substituenten aus der Gruppe Nitro, Cyano, Azido, Hydroxy, Amino, Carboxy, Halogen, Phenyl, Alkyl, Alkoxy, Trifluormethyl, Trifluormethoxy, Benzoyloxy, Alkanoyloxy, Trifluormethylthio, Alkylmercapto, Alkylsulfonyl oder Alkylamino substituiert sind, wobei die genannten Alkyl- und Alkoxyreste jeweils 1 bis 4 Kohlenstoffatome enthalten, und ihrerseits substituiert sind durch Hydroxy, Alkoxy, Alkanoyloxy mit jeweils 1 bis 4 C-Atomen, Benzoyloxy oder die

Gruppe 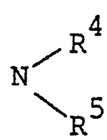 , mit den unten angegebenen Bedeutungen für $R^4$ und $R^5$,

m und n gleich oder verschieden sind und jeweils 0 oder 1 bedeuten und

Y für Sauerstoff, Schwefel oder die Gruppe NH steht, und

$R^3$ für geradkettiges oder verzweigtes Alkyl oder Alkenyl mit bis zu 6 Kohlenstoffatomen steht, gegebenenfalls substituiert durch Hydroxy, Alkoxy, (1-4 C-Atome), Fluor, Chlor, Brom, Phenyl, Furyl, Carboxy, Carbalkoxy oder die

Gruppe 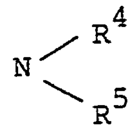, mit den unten angegebenen Bedeutungen für $R^4$ und $R^5$, oder für Phenyl, Furyl, Pyridyl oder Naphthyl steht, wobei diese aromatischen Ringe gegebenenfalls 1 oder 2 gleiche oder verschiedene Substituenten aus der Gruppe Cyano, Hydroxy, Amino, Carboxy, Halogen, Phenyl, Alkyl, Alkoxy, Trifluormethyl, Trifluormethoxy, Trifluormethylthio, Alkylamino trägt, wobei die genannten Alkyl- und Alkoxyreste jeweils 1 bis 4 Kohlenstoffatome enthalten, steht oder

$R^3$ Wasserstoff bedeutet, wenn

a) R und $R^1$ gemeinsam einen Ring bilden,

b) $R^1$ oder $R^2$ Wasserstoff oder gegebenenfalls substituiertes Alkyl bedeuten, oder

c) $R^1$ und $R^2$ für $R^{1'}$ und $R^{2'}$ stehen, wobei mindestens einer dieser Reste Phenyl bedeutet, welches 1 oder 2 gleiche oder verschiedene Substituenten aus der Gruppe Acyl mit 1 bis 4 Kohlenstoffatomen, Hydroxyalkyl, Hydroxyalkoxy mit jeweils 1 bis 4 Kohlenstoffatomen, wobei die Hydroxylgruppen gegebenenfalls durch

Benzoyl oder Alkanoyl mit 1 bis 4 Kohlenstoffatomen substituiert sind, oder die Gruppe $NR^{4'}R^{5'}$, wobei $R^{4'}$ und $R^{5'}$ gemeinsam mit dem Stickstoffatom einen 4- bis 7-gliedrigen Ring bilden, der gegebenenfalls durch Sauerstoff, Schwefel, die NH-Gruppe, die N-Alkyl(1-4 C-Atome)-Gruppe oder die N-Phenylgruppe unterbrochen ist, und

$R^4$ und $R^5$ gleich oder verschieden sind und jeweils für Wasserstoff, Benzyl, Alkyl oder Alkenyl mit bis zu 6 Kohlenstoffatomen stehen, wobei die Alkyl- und Benzylreste gegebenenfalls substituiert sind durch die Gruppe $COR^5$, wobei $R^5$ Alkyl mit 1 bis 4 Kohlenstoffatomen, Benzyl oder Phenyl bedeutet oder

$R^4$ und $R^5$ gemeinsam mit dem Stickstoffatom einen 5- bis 7-gliedrigen Ring bilden, der gegebenenfalls durch Sauerstoff, Schwefel, NH oder $NR^6$ unterbrochen ist, wobei $R^6$ die oben angegebene Bedeutung besitzt.

Besonders hervorgehoben seien Verbindungen der allgemeinen Formel (I), in welcher

R für Wasserstoff, Alkyl mit 1 bis 4 Kohlenstoffatomen, Benzyl oder Phenyl steht, oder in welcher

R und $R^1$ einen carbocyclischen Ring mit 3 bis 6 Kohlenstoffatomen formen, an welchen ein Benzolring ankondensiert ist,

$R^1$ und $R^2$ gleich oder verschieden sind und jeweils für Wasserstoff, Alkyl mit 1 bis 4 Kohlenstoffatomen, Pyridyl, Furyl oder für Phenyl stehen, wobei der Phenylring gegebenenfalls 1 oder 2 gleiche oder verschiedene Substituenten aus der Gruppe Fluor, Chlor, Nitro, Cyano, Amino, Azido, Trifluormethyl, Trifluormethoxy, Trifluormethylthio, Alkylmercapto und Alkoxy mit 1 bis 4 Kohlenstoffatomen je Alkylrest, N-Pyrrolidino, N-Piperidino, N-Phenylpiperazino, N-Morpholino, Hydroxymethyl, N-Pyrrolidinomethyl, N-Piperidinomethyl, N-Phenylpiperazinomethyl, N-Morpholinomethyl, Benzoyloxymethyl, Alkanoyloxymethyl, wobei die Alkylgruppe 1 bis 6 Kohlenstoffatomen haben kann und ihrerseits substituiert sein kann durch Carboxy oder Carbalkoxy trägt,

m und n 0 oder 1 sein können,

Y für Sauerstoff, Schwefel oder die Gruppe NH steht,

$R^3$ nur dann Wasserstoff sein darf, wenn

a) R und $R^1$ einen Ring formen oder

b) $R^1$ oder $R^2$ für Wasserstoff oder Alkyl stehen, oder wenn

c) $R^1$ und $R^2$ für $R^{1'}$ und $R^{2'}$ stehen, wobei mindestens einer der Substituenten Phenyl bedeutet, welches substituiert ist durch 1 oder 2 gleiche oder verschiedene Substituenten aus der Gruppe Acyloxy mit 2 oder 3 C-Atomen, Hydroxyalkyl mit 1 bis 4 C-Atomen, wobei die Hydroxygruppe gegebenenfalls durch Benzoyl oder Alkanoyl mit 1 bis 6 C-Atomen substituiert ist, wobei der Alkanoylrest seinerseits wieder eine Carboxy oder Carbalkoxy mit 2 bis 4 C-Atomen enthalten kann, N-Pyrrolidonomethyl, N-Piperidinomethyl oder N-Morpholinomethyl,

und im übrigen

$R^3$ für geradkettiges oder verzweigtes Alkyl bzw. Alkenyl mit bis zu 6 Kohlenstoffatomen steht, welches gegebenenfalls substituiert ist durch Hydroxy, Alkoxy, Fluor, Chlor, Brom, Phenyl, Carboxy, Carbalkoxy, N-Pyrrolidono, N-Piperidino, N-Morpholino,
oder für Phenyl steht, welches gegebenenfalls substituiert ist durch Fluor, Chlor, Brom, Hydroxy, Alkoxy und Alkyl mit 1 bis 4 Kohlenstoffatomen, Amino, Alkylamino und Dialkylamino mit je 1 bis 4 Kohlenstoffatomen, Trifluormethyl, Cyano, Nitro oder Carboxy, und

$R^4$ und $R^5$ gleich oder verschieden sind und für Alkyl mit 1 bis 4 Kohlenstoffatomen oder Benzyl stehen oder

$R^4$ und $R^5$ gemeinsam mit dem Stickstoffatomen einen 5- oder 6-gliedrigen Ring bilden, der gegebenenfalls durch die Gruppe NH oder $NR^6$ unterbrochen ist, wobei $R^6$ die oben angegebene Bedeutung besitzt.

Überraschenderweise zeigen die erfindungsgemäßen Verbindungen der Formel (I) eine sehr starke Wirkung bei der Behandlung von Fettstoffwechselstörungen. Sie bewirken insbesondere eine Senkung des erhöhten Cholesterins im Serum und vermindern gleichzeitig eine Hypertriglyceridämie.

Die erfindungsgemäßen Verbindungen eignen sich daher vorteilhaft zur Behandlung von Hyperlipoproteinämien, Atherosklerose, Adipositas und zur Behandlung hierdurch ausgelöster Stoffwechselstörungen.

Die erfindungsgemäßen Verbindungen sind besonders geeignet als Lipidabsorptionshemmer, Diuretika, Saluretika, Antiarrythmika und Cardiotonika.

Die diuretische und saluretische Wirkung wird an Ratten untersucht. Hierzu werden männliche Ratten verwendet, die in nüchternem Zustans 10 ml/kg Flüssigkeit per Schlundsonde erhalten. Diese Flüssigkeit enthält 0,5 % Tylose sowie eine bestommte Dosis des Prüfpräparates (Kontrolltiere ohne Prüfpräparat). Der ausgeschiedene Harn wird 6 Stunden gesammelt und anschließend der Natrium- und Kaliumgehalt in üblicher Weise photometrisch bestimmt.

Die antiarrythmische Wirkung der erfindungsgemäßen Verbindungen wird durch die Einflüsse auf die Refraktärzeit der Herzmuskulatur mittels Standardtestverfahren nachgewiesen. Bekanntlich verlängern Antiarrythmika in therapeutischen Dosen die Refraktärzeit der Herzmuskulatur. Diese Verlängerung der Refraktärzeit sowie die Bestimmung der Kontraktionskraft an isolierten Herzmuskel-Präparaten erfolgt nach bekannten Methoden (vgl.: Govier, J. Pharmacol. Exp. Ther. 148, 100-105, (1965) und Roseblueth et al., J. Cell Comp. Physiol. 33, 405-439 (1949)).

Cholesterol- bzw. Fettbelastungstests wurden an nüchternen männlichen oder weiblichen Wistar-Ratten mit einem Körpergewicht zwischen 130-230 g durchgeführt.

Cholesterol-Belastung:

Cholesterol und die zu prüfende Substanz wurden in 0,75 %iger Traganth-Suspension gleichzeitig oral appliziert. Nach 24 h wurden die Tiere getötet und der Cholesterolgehalt in der Leber bestimmt. Cholesterol wurde nach der Liebermann-Burchard-Methode mit der Testkombination von Boehringer Mannheim bestimmt. Kontrolltiere erhielten ein entsprechendes Volumen Cholesterol-Traganth-Suspension ohne Testsubstanz. Zur Kontrolle des Cholesterol-Anstiegs als Folge der Cholesterol-Belastung wurde einer weiteren Kontrollgruppe Traganth-Suspension ohne Cholesterol oral appliziert.

Fettbelastung:

Olivenöl und die zu prüfende Substanz wurden in 0,75 %-iger Traganth-Suspension gleichzeit oral appliziert. Nach 1 h bis 6 h wurde den Tieren per Orbitalpunktion Blut entnommen und der Triglyceridgehalt im Serum enzymatisch nach der kinetischen Methode mit der Testkombination "Triglyceride vollenzymatisch" von Boehringer Mannheim bestimmt.

Kontrolltiere erhielten ein entsprechendes Volumen Olivenöl-Traganth-Suspension ohne Testsubstanz. Zur Kontrolle des Triglyceridanstiegs im Serum als Folge

der Olivenölbelastung wurde einer weiteren Kontrollgruppe Traganth-Suspension ohne Olivenöl oral appliziert.

Die überraschende Wirkung der erfindungsgemäßen Stoffgruppe sei beispielhaft durch die folgenden Testergebnisse belegt.

| Beispiel-Nr. | Dosis (mg/kg) p.o. | Verminderung des Lebercholesterinanstiegs im Vergleich zur Kontrolle nach oraler Cholesterinbelastung in Prozent |
|---|---|---|
| 1 | 10 | 51,7 % |
| 4 | 10 | 31,9 % |
| | 30 | 58,6 % |
| 8 | 10 | 21,3 % |
| 9 | 10 | 17,7 % |
| 13 | 10 | 37,9 % |
| 16 | 10 | 36,7 % |
| 17 | 10 | 22,9 % |
| 18 | 10 | 34,1 % |
| 19 | 10 | 36,3 % |
| 21 | 10 | 34,9 % |
| 25 | 10 | 22,6 % |
| 29 | 10 | 12,0 % |
| 34 | 10 | 23,9 % |

Es ist als ausgesprochen überraschend zu bezeichnen, daß die erfindungsgemäßen Verbindungen diese neuen und vorteilhaften Wirkungen besitzen. Als neuartige Stoffklasse zur Behandlung von Stoffwechselstörungen und Herzrhythmusstörungen, bei gleichzeitig sehr guter Verträglichkeit, stellen sie eine Bereicherung der Pharmazie dar.

Zur vorliegenden Erfindung gehören pharmazeutische Zubereitungen, die neben nichttoxischen, inerten pharmazeutisch geeigneten Trägerstoffen eine oder mehrere Verbindungen der obigen Formel enthalten oder die aus einer oder mehreren Verbindungen der obigen Formel bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen, vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum-, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Lösungen und Emulsionen können neben dem oder den Wirkstoffen die üblichen Trägerstoffe, wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.
Suspensionen können neben dem oder den Wirkstoffen die üblichen Trägerstoffe, wie flüssige Verdünnungsmittel, z.B. Wasser, Ethylalkohol, Propylenglykol, Suspendiermittel z.B. ethoxylierte Isostearylalkohole, Polyoxyethylensorbit- und sorbitanester, mikrokristalline Cellulose, Aluminiummethanhydroxid, Bentonit, Agar-Agar und Traganth oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer Verbindungen der obigen Formel auch andere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der Verbindungen der obigen Formel sowie die Verwendung von pharmazeutischen Zubereitungen, die eine oder mehrere Verbindungen der oben angegebenen Formel enthalten, in der Human- und Veterinärmedizin zur Verhütung, Besserung und/oder Heilung der oben angeführten Erkrankungen.

Die Wirkstoffe oder die pharmazeutischen Zubereitungen können oral, parenteral, intraperitoneal und/oder rektal, vorzugsweise oral, appliziert werden.

Im allgemeinen hat es sich sowohl in der Human- als auch in der Veterinärmedizin als vorteilhaft erwiesen, den oder die Wirkstoffe in Mengen von etwa 1,0 bis etwa 500, vorzugsweise 5 bis 100 mg/kg Körpergewicht je 24 Stunden, verteilt auf 1 bis 6 Verabreichungen und zwar vor oder/und während oder/ und nach der Mahlzeit zu applizieren. Eine Einzelgabe enthält den oder die Wirkstoffe, vorzugsweise in Mengen von etwa 1 bis etwa 100 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der o.g. Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiele

Die Beispiele 1 bis 9 illustrieren die Herstellung der Zwischenprodukte der allgemeinen Formel (IV)

Beispiel 1

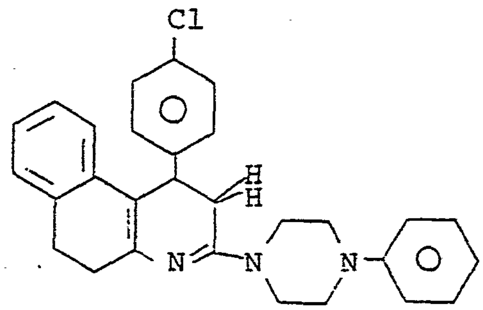

11,8 g 1-(4-Chlorbenzyliden)-2-tetralon werden zusammen mit 12,0 g 3-Amino-3-(4-phenylpiperazino)-acrylsäure-ethylester in 350 ml Ethanol 4 h unter Rückfluß erhitzt. Dann wird das Ethanol abdestilliert, der Rückstand in Methylenchlorid gelöst und 2 mal mit verdünnter Schwefelsäure extrahiert. Die Methylenchloridphase wird eingedampft, auf den Rückstand Ether gegeben und gerade soviel Methanol, um eine klare Lösung zu erzielen. Die etherische Lösung wird 3 mal mit verdünnter Schwefelsäure gewaschen, die sauren wäßrigen Phasen mit festem Natriumcarbonat basisch gestellt und 2 mal mit Methylenchlorid extrahiert. Die vereinigten organischen Phasen werden mit Natriumsulfat getrocknet und im Vakuum eingedampft. Der Rückstand kristallisiert aus Ethanol. Nach Absaugen des Niederschlags kristallisiert man aus Ethanol um und erhält so 9,3 g 4-(4-Chlorphenyl)-2-(4-phenylpiperazino)-3,4,5,6-tetrahydronaphthalino(a)pyridin.
Ausbeute: 47 % der theoretischen Ausbeute
Schmelzpunkt: 134-135°C

Die in den Tabellen 1 und 2 aufgeführten Beispiele wurden analog zu Beispiel 1 hergestellt.

Tabelle 1

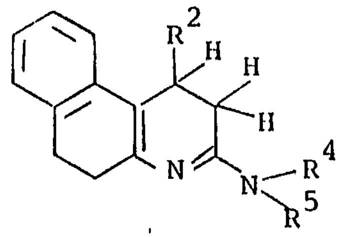

| Bsp. Nr. | $R^2$ | $R^4$ | $R^5$ | Umkristallisiert aus Lösungsmittel | Fp °C | Ausbeute % d.Th. |
|---|---|---|---|---|---|---|
| 2 | 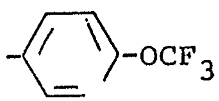 | $(-C_2H_4)_2N-$ | 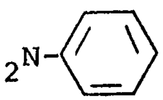 | Ethanol | 155 | 65 |
| 3 | 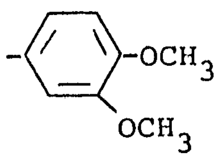 | $(-C_2H_4)_2N-$ | 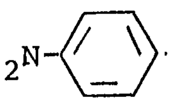 | Cyclohexan | 63 | 42 |
| 4 | 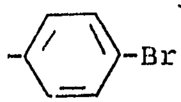 | $(-C_2H_4)_2N-$ | 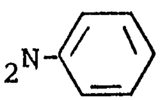 | Methanol | 143 | 37 |
| 5 | 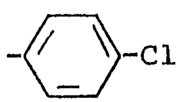 | $-(CH_2)_4-$ | | Methanol | 189 | 30 |

Tabelle 2

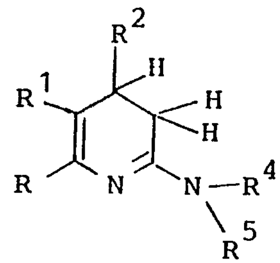

| Bsp. Nr. | R | $R^1$ | $R^2$ | $R^4$ | $R^5$ | Umkristallisation aus Lösungsmittel | Fp. °C | Ausbeute % d. Th. |
|---|---|---|---|---|---|---|---|---|
| 6 | $CH_3$ | 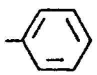 | 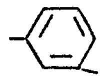 $-CH_2OOCCH_3$ | $(-C_2H_4)_2N-$ | 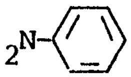 | Ether, x 2 HCl | 65 | 57 |
| 7 | $CH_3$ | 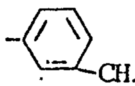 $-CH_2OOCCH_3$ | 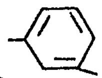 $-CH_2OOCCH_3$ | $(-C_2H_4)_2N-$ | 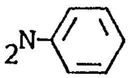 | Ether, x 2 HCl | 63 | 48 |
| 8 | 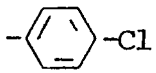 $-Cl$ | H | 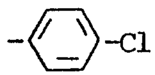 $-Cl$ | $(-C_2H_4)_2N-$ | 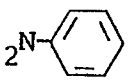 | Aceton, x 2 HCl | 152-154 | 7 |
| 9 | $CH_3$ | 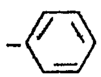 $-CH_2OOCCH_3$ | 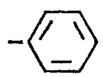 $-CH_2OOCCH_3$ | $(-C_2H_4)_2N-$ | 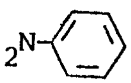 | Ether, x 2 HCl | 80-85 | 12 |

Beispiel 10

9,4 g (0,02 Mol) 4,5-Bis-(4-methoxyphenyl)-6-methyl-2-(4-phenylpiperazino)-3,4-dihydropyridin werden in 250 ml Dichlormethan gelöst und bei -20°C mit 64 ml einer Lösung von 100 ml Bortribromid in 300 ml Dichlormethan tropfenweise versetzt. Man rührt 1 h nach und tropft dann langsam 100 ml Methanol so zu, daß die Temperatur 0°C nicht übersteigt. Die Reaktionslösung wird dann auf ges. Bicarbonatlösung gegossen und mehrmals mit Dichlormethan extrahiert. Nach Trocknen über Natriumsulfat und Eindampfen kristallisiert man den so erhaltenen Rückstand aus Ether/Methanol um. Man erhält so 6,6 g 4,5-Bis-(4-hydroxyphenyl)-6-methyl-2-(4-phenylpiperazino)-3,4-dihydropyridin mit einem Zersetzungspunkt von 185-186°C. Diese werden in 140 ml Dichlormethan gelöst und bei 0°C mit 2 g 1,5-Diazabicyclo(4,3,0)-non-5-en und 3,5 g Acetylchlorid versetzt. Nach 3-stündigem Rühren bei Raumtemperatur wird mehrmals mit ges. Bicarbonatlösung extrahiert, mit Natriumsulfat getrocknet und eingedampft. Umkristallisieren des Rückstands aus Ether ergibt 2 g 4,5-Bis-(4-acetoxyphenyl)-6-methyl-2-(4-phenylpiperazino)-3,4-dihydropyridin.
Ausbeute: 87 % der theoretischen Ausbeute
Schmelzpunkt: 141-142°C.

Beispiel 11

35,7 g (0,015 Mol) 4-(3-Acetoxymethylphenyl)-6-methyl-5-phenyl-2-(4-phenylpiperazino)-3,4-dihydropyridin (siehe Beispiel Nr. 6) wurden in 300 ml absolutem Methanol mit 1 g Natriummethylat versetzt und 3 h bei Raumtemperatur stehen gelassen. Nach dem Eindampfen kristallisierte der Rückstand durch. Nach Verrühren mit Ether erhält man 17 g weiße Kristalle von 4-(3-Hydroxymethylphenyl)-6-methyl-5-phenyl-2-(4-phenylpiperazino)-3,4-dihydropyridin.
Ausbeute: 52 %,
Schmelzpunkt: 137-139°C.

Die in Tabelle 3 aufgeführten Beispiele wurden analog zu Beispiel 11 aus den entsprechenden Acetaten hergestellt.

Tabelle 3

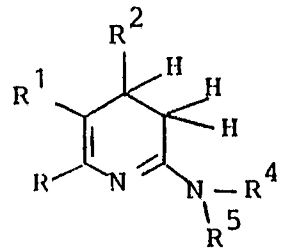

| Bsp. Nr. | R | $R^1$ | $R^2$ | $R^4$ $R^5$ | Umkrist. aus Lösungs-mittel | Fp. °C | Ausbeute % d. Th. |
|---|---|---|---|---|---|---|---|
| 12 | $CH_3$ | 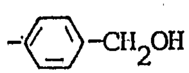 | 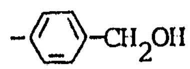 | $(-C_2H_4)_2N-$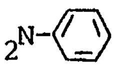 | Methanol | 178-180 | 46 |
| 13 | $CH_3$ | 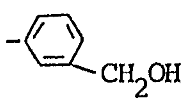 | 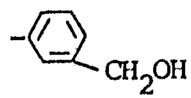 | $(-C_2H_4)_2N-$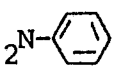 | Ether | 61-63 | 51 |
| 14 | $CH_3$ | 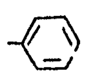 | 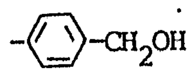 | $-(C_2H_4)_2N-$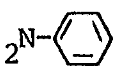 | Ether, x 2 HCl | 95-100 | 30 |
| 15 | $CH_3$ | 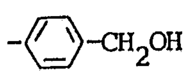 | 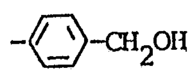 | $-(CH_2)_4-$ | Ether/Methanol | 103-105 Zers. | 57 |
| 16 | $CH_3$ | 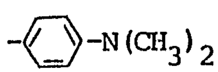 | 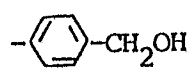 | $(-C_2H_4)_2N-$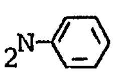 | Ether | 142-144 | 8 |

Beispiel 17

0,437 g 4-(3-Hydroxymethylphenyl)-6-methyl-5-phenyl-2-(4-phenylpiperazino)-3,4-dihydropyridin (siehe Beispiel 11) werden in 5 ml absolutem Methylenchlorid gelöst und bei 0°C unter Rühren mit 0,1 ml Phosphortribromid versetzt. Nach 2 stündigem Rühren bei dieser Temperatur wird mit 50 ml Methylenchlorid verdünnt und 2 x mit ges. Bicarbonatlösung extrahiert. Die Methylenchloridphase wird dann über Natriumsulfat getrocknet und im Vakuum eingedampft. Der Rückstand wird in 10 ml abs. Methylenchlorid gelöst und mit 0,5 ml Pyrrolidin versetzt und 2 h bei Raumtemperatur stehen gelassen. Anschließend wird mit 50 ml Methylenchlorid verdünnt und 2 x mit ges. Bicarbonatlösung extrahiert. Die Methylenchloridphase wird über Natriumsulfat getrocknet und im Vakuum eingeengt. Der Rückstand wird in Ether suspendiert, filtriert und das Filtrat mit etherischer Salzsäure versetzt. Vom so ausgefällten Hydrochlorid wird abdekantiert, der Rückstand in Methanol gelöst und im Vakuum eingedampft. Aus Cyclohexan erhält man 0,4 g kristallines, 6-Methyl-5-phenyl-2-(4-phenylpiperazino)-4-(3-pyrrolidinomethylphenyl)-3,4-dihydropyridin.
Ausbeute: 67 % der theoretischen Ausbeute
Schmelzpunkt: 90-92°C x 3 HCl

Beispiel 18

Analog zu Beispiel 17 wird 6-Methyl-2-(4-phenylpiperazino)-4,5-bis(2-pyrrolidinomethylphenyl)-3,4-dihydropyridin aus 4,5-Bis-(3-hydroxymethylphenyl)-6-methyl-2-(4-phenylpiperazino)-3,4-dihydropyridin (siehe Beispiel 17) hergestellt und aus Cyclohexan als Hydrochlorid kristallisiert.
Ausbeute: 42 % der theoretischen Ausbeute
Schmelzpunkt: 69-70°C x 4 HCl

Beispiel 19

2,2 g 4-(3-Hydroxymethylphenyl)-6-methyl-5-phenyl-2-(4-phenylpiperazino)-3,4-dihydropyridin (siehe Beispiel 11) werden in 5 ml absolutem Dimethylformamid zusammen mit 0,66 g Glutarsäureanhydrid 12 h bei 80°C gerührt. Dann wird im Hochvakuum das Lösungsmittel abgezogen und der Rückstand an Kieselgel 60 (0,063-0,2 mm) mit Methylenchlorid/Methanol 10:1 als Laufmittel chromatographiert. Man erhält so neben Ausgangsmaterial 0,5 g 4-(3-(5-Carboxybutyryloxymethyl)-phenyl)-6-methyl-5-phenyl-2-(4-phenylpiperazino)-3,4-dihydropyridin, das aus Cyclohexan 0,4 g kristallines Material liefert.
Ausbeute: 14 % der theoretischen Ausbeute
Schmelzpunkt: 70-72 °C

Beispiel 20

1,4 g 4-(4-Chlorphenyl)-5-(4-dimethylaminophenyl)-6-methyl-2-(4-phenylpiperazino)-3,4-dihydropyridin werden bei -78°C zu einer Lösung von 0,96 g Lithiumdiisopropylamid in 20 ml absolutem Tetrahydrofuran unter Stickstoff gegeben. Nach Erwärmen der Reaktionslösung auf -10°C und Erhalt einer klaren, gelborangenen Lösung kühlt man wieder auf -78°C und gibt tropfenweise 1,5 g Benzylbromid zu der heftig gerührten Reaktionslösung. Nach 30 minütigem Rühren bei -78°C wird zunächst tropfenweise 2n Schwefelsäure zugegeben, dann mit 150 ml 2n Schwefelsäure verdünnt und 2 x mit Methylenchlorid extrahiert. Die saure, wäßrige Phase wird mit festem Natriumcarbonat alkalisch gestellt und mit Methylenchlorid 3 x extrahiert.

Nach Trocknen mit Natriumsulfat und Eindampfen kristallisiert der Rückstand aus einem 1:1-Gemisch von Ether mit Petrolether. Nach Umkristallisation in obigem Lösungsmittelgemisch erhält man 1 g 3-Benzyl-4-(4-chlorphenyl)-5-(4-dimethylaminophenyl)-6-methyl-2-(4-phenylpiperazino)-3,4-dihydropyridin.
Ausbeute: 58 % der theoretischen Ausbeute
Schmelzpunkt: 180-182°C.

Die in Tabelle 4 aufgeführten Beispiele werden analog zu Beispiel 20 durch Reaktion der jeweiligen 4,5-Diaryl-3,4-dihydropyridinderivate mit den entsprechenden Elektrophilen hergestellt:

Tabelle 4

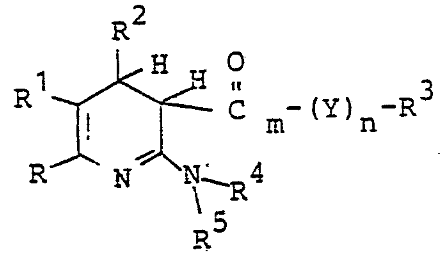

| Bsp. Nr. | R | $R^1$ | $R^2$ | m | n | y | $R^3$ |
|---|---|---|---|---|---|---|---|
| 21 | $CH_3$ | 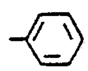 | 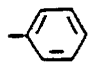 | 0 | 0 | - | $-CH_2CH=CH_2$ |
| 22 | $CH_3$ | 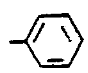 | 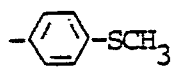 | 0 | 0 | - | $-CH_2-$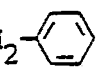 |
| 23 | $CH_3$ | 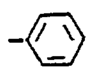 | 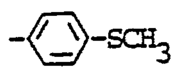 | 0 | 0 | - | $-CH_2-COOC(CH_3)_3$ |
| 24 | $CH_3$ | 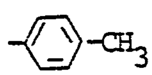 | 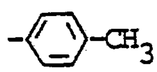 | 0 | 0 | - | $-CH_3$ |
| 25 | $CH_3$ | 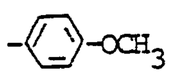 | 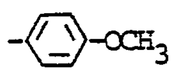 | 0 | 0 | - | $-CH_3$ |
| 26 | $CH_3$ | 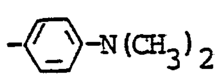 | 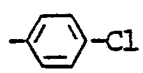 | 0 | 0 | - | $-CH_3$ |
| 27 | $CH_3$ | 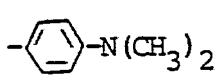 | 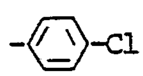 | 0 | 0 | - | $-CH_2-CH=CH_2$ |
| 28 | $CH_3$ | 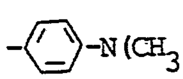 | 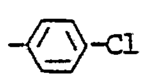 | 0 | 0 | - | $-CH_2-$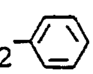 |
| 29 | $CH_3$ | 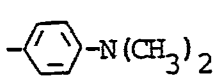 | 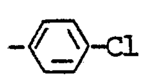 | 0 | 0 | - | $-CH_2COOC(CH_3)_3$ |
| 30 | $CH_3$ | 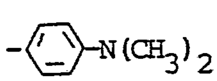 | 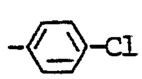 | 0 | 0 | - | $-CH_2OH$ |
| 31 | $CH_3$ | 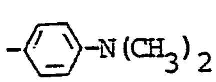 | 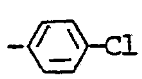 | 1 | 1 | O | $-C_2H_5$ |
| 32 | $CH_3$ | 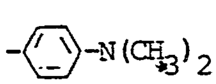 | 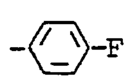 | 0 | 1 | S | 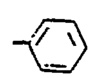 |

Tabelle 4 (Fortsetzung)

| Bsp. Nr. | $R^4$ | $R^5$ | Umkrist. aus Lösungsmittel | Fp. °C | Ausbeute % d.Th. | Elektrophit |
|---|---|---|---|---|---|---|
| 21 | $(-C_2H_4)_2N-$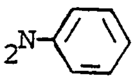 | | Aceton, x 2 HCl | 209-211 | 32 | Allylbromid |
| 22 | $(-C_2H_4)_2N-$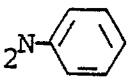 | | Aceton, x 2 HCl | 172-175 | 16 | Benzylbromid |
| 23 | $(-C_2H_4)_2N-$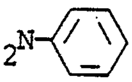 | | Ether, x 2 HCl | 88-91 | 26 | Bromessigsäure-tert.-butylester |
| 24 | $(-CH_2)_4-$ | | Ether | 166-167 | 80 | Methyljodid |
| 25 | $(CH_2)_4-$ | | Ether/Petrolether | 110-111 | 63 | Methyljodid |
| 26 | $(-C_2H_4)_2N-$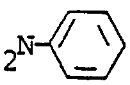 | | Ether/Petrolether | 175 | 50 | Methyljodid |
| 27 | $(-C_2H_4)_2N-$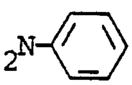 | | Aceton, x 3 HCl | 186-188 | 37 | Allylbromid |
| 28 | $(-C_2H_4)_2N-$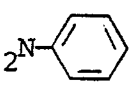 | | Ether/Petrolether | 180-182 | 58 | Benzylbromid |
| 29 | $(-C_2H_4)_2N-$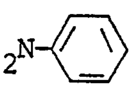 | | Ether/Petrolether | 59-62 | 31 | Bromessigsäure-tert.-butylester |
| 30 | $(-C_2H_4)_2N-$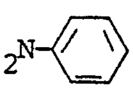 | | Ether/Petrolether | 77-80 | 68 | Paraformaldehyd |
| 31 | $(-C_2H_4)_2N-$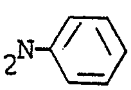 | | Ether/Petrolether | 60-63 | 36 | Pyrokohlensäurediethylester |
| 32 | $(-C_2H_4)_2N-$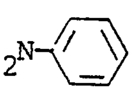 | | Ether | 160 | 70 | Diphenyldisulfid |

Tabelle 4 (Fortsetzung)

| Bsp. Nr. | R | $R^1$ | $R^2$ | m | n | y | $R^3$ |
|---|---|---|---|---|---|---|---|
| 33 | $CH_3$ | 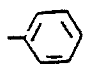 | 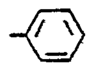 | 1 | 1 | O | $-C_2H_5$ |
| 34 | $CH_3$ | 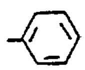 | 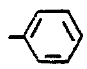 | o | o | - | $-CH_2OH$ |
| 35 | $CH_3$ | 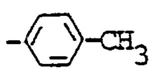 | 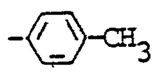 | 1 | 1 | O | $-C_2H_5$ |
| 36 | $CH_3$ | 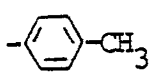 | 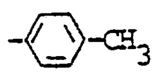 | o | o | - | $-CH_2OH$ |
| 37 | $CH_3$ | 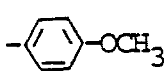 | 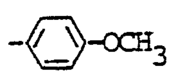 | o | o | - | $-CH_2OH$ |
| 38 | $CH_3$ | 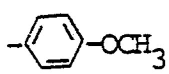 | 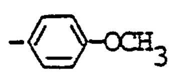 | 1 | 1 | O | $-C_2H_5$ |

Tabelle 4 (Fortsetzung)

| Bsp. Nr. | $R^4$ | $R^5$ | Umkrist. aus Lösungsmittel | Fp. °C | Ausbeute % d.Th. | Elektrophil |
|---|---|---|---|---|---|---|
| 33 | $(-C_2H_4)_2N-$ 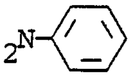 | | Ether, x 2 HCl | 190-192 | 20 | Pyrokohlensäure-diethylester |
| 34 | $(-C_2H_4)_2N-$ 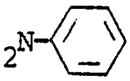 | | Ether/Petrol-ether | 126-127 | 15 | Paraformaldehyd |
| 35 | $-(CH_2)_4-$ | | Ether/Petrol-ether | 143-145 | 69 | Pyrokohlensäure-diethylester |
| 36 | $-(CH_2)_4-$ | | Cyclohexan/Petrolether | 130-131 | 71 | Paraform-aldehyd |
| 37 | $-(CH_2)_4-$ | | Ether | 134-135 | 40 | Paraform-aldehyd |
| 38 | $-(CH_2)_4-$ | | Ether/Petrol-ether | 137-138 | 68 | Pyrokohlen-säurediethyl-ester |

Patentansprüche

1) Substituierte 2-Amino-3,4-dihydropyridinderivate der allgemeinen Formel (I)

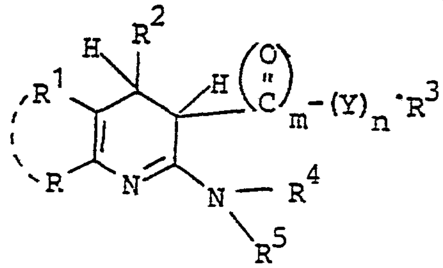

(I)

in welcher

R für Wasserstoff, Alkyl, gegebenenfalls substituiert durch Hydroxy, Halogen, Alkoxy, Aryl oder die Gruppe

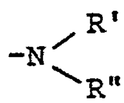 steht,

wobei R' und R" gleich oder verschieden sind und jeweils Wasserstoff, Alkyl oder Aralkyl bedeuten, oder für gegebenenfalls substituiertes Aralkyl steht, oder für Aryl steht, welches gegebenenfalls durch 1 oder 2 gleiche oder verschiedene Substituenten aus der Gruppe Halogen, Alkyl, Alkoxy, Alkylmercapto oder Trifluormethyl substituiert ist, oder

R und $R^1$ gemeinsam gegebenenfalls einen carbocyclischen Ring bilden, der gegebenenfalls substituiert ist durch Hydroxy, Halogen, Nitro, Alkyl, Alkoxy oder die

Gruppe 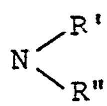 , wobei R' und R" die oben angeführten Bedeutungen haben, und wobei an den carbocyclischen Ring ein Arylring ankondensiert sein kann, der gegebenenfalls substituiert ist durch 1 oder 2 gleiche oder verschiedene Substituenten aus der Gruppe Halogen, Alkyl, Alkoxy, Alkylmercapto oder Trifluormethyl, und wobei der Arylring gegebenenfalls durch Sauerstoff, Schwefel oder die $NR^6$-Gruppe unterbrochen ist, wobei $R^6$ die für $R^1$ und $R^2$ angegebene Bedeutung besitzt,

$R^1$ und $R^2$ gleich oder verschieden sind und jeweils für Wasserstoff oder Alkyl, gegebenenfalls substituiert durch Aryl, Heteroaryl, Hydroxyl, Halogen, Alkoxy, Carboxy, Cyano, Carbalkoxy oder die

Gruppe 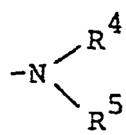 mit den unten angeführten Bedeutungen für $R^4$ und $R^5$ stehen,

oder

für einen Arylrest oder für einen Heteroarylrest aus der Gruppe Thienyl, Furyl, Pyrryl, Pyrazolyl, Imidazolyl, Oxazolyl, Isooxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl stehen, wobei der Arylrest sowie die Heteroarylreste gegebenenfalls 1, 2 oder 3 gleiche oder verschiedene Substituenten aus der Gruppe

Phenyl, Alkyl, Alkenyl, Alkinyl, Alkoxy, Alkenoxy, Alkinoxy, Alkylen, Dioxyalkylen, Halogen, Acyloxy, Trifluormethylthio, Trifluormethyl, Trifluormethoxy, Hydroxy,

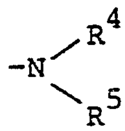 ,

mit den unten angegebenen Bedeutungen für $R^4$ und $R^5$, Nitro, Cyano, Azido, Carboxy, Carbalkoxy, Carbonamido, Sulfonamido oder $SO_{m'}$-Alkyl (m' = 0, 1 oder 2) enthalten, und wobei die Alkyl- und Alkoxysubstituenten ihrerseits gegebenenfalls substituiert sind durch Hydroxy, Alkoxy, Carboxy, Carbalkoxy, Halogen, die

Gruppe 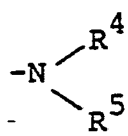 ,

mit den unten angegebenen Bedeutungen für $R^4$ und $R^5$, Aroyloxy oder Alkanoyloxy, wobei der Aroyl- und Alkanoylrest durch Hydroxy, Alkoxy, Halogen, Carboxy oder Carbalkoxy substituiert sein kann,

m und n gleich oder verschieden sind und jeweils 0 oder 1 bedeuten,

Y für Sauerstoff, die Gruppe $SO_m-R^6$, (m' = 0, 1, 2) oder die Gruppe $N-R^6$, wobei $R^6$ einen Alkyl- oder Arylrest bedeutet, steht, und

$R^3$ für Alkyl, gegebenenfalls substituiert durch Hydroxy, Alkoxy, Halogen, die Gruppe

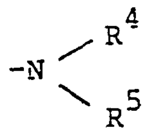

, mit den unten angeführten Bedeutungen für $R^4$ und $R^5$, Carboxy, Carbalkoxy, Cyano, Alkenyl oder Aryl steht,

oder

für Aryl, welches gegebenenfalls substituiert ist durch 1 oder 2 gleiche oder verschiedene Substituenten der Gruppe Hydroxy, Alkoxy, Halogen, Alkyl, Alkenyl, Aryl, Alkylmercapto, Alkylamino, Carboxy, Carbalkoxy, Cyano, Trifluormethyl, Trifluormethoxy, Trifluormethylthio, steht

oder

$R^3$ auch für Wasserstoff steht, wenn

a) R und $R^1$ gemeinsam einen Ring bilden oder

b) $R^1$ und $R^2$ für Wasserstoff oder gegebenenfalls substituiertes Alkyl stehen, oder

c) $R^1$ und $R^2$ für $R^{1'}$ und $R^{2'}$ stehen, wobei mindestens einer dieser Substituenten für Aryl steht, welches 1, 2 oder 3 gleiche oder verschiedene Substituenten aus der Gruppe Acyloxy, Arylmercapto, Hydroxyalkyl, Hydroxyalkoxy, Hydroxyalkylmercapto und $NR^{4'}R^{5'}$ trägt,

wobei

die Hydroxylgruppen der Alkyl- und Alkoxysubstituenten gegebenenfalls ihrerseits substituiert sind durch Aroyl oder Alkanoyl, die ihrerseits wieder eine Carboxy- oder Carbalkoxygruppe als Substituenten tragen können, und

wobei
$R^{4'}$ und $R^{5'}$ mit dem Stickstoffatom einen 4- bis 7-gliedrigen Ring bilden, der gegebenenfalls durch Sauerstoff, Schwefel, die NH-Gruppe oder die $NR^6$-Gruppe unterbrochen ist, wobei $R^6$ die oben angegebene Bedeutung besitzt, und

$R^4$ und $R^5$ gleich oder verschieden sind und jeweils für Wasserstoff oder für einen Alkyl-, Aralkyl-, Alkenyl- oder Alkinylrest stehen, wobei die Alkyl- und Alkenylreste geradkettig, verzweigt oder cyclisch sind und gegebenenfalls substituiert sind durch die Gruppe $COR^5$, wobei $R^5$ Alkyl, Aralkyl oder Aryl bedeutet,
oder

$R^4$ und $R^5$ gemeinsam mit dem Stickstoffatom einen 4- bis 7-gliedrigen Ring bilden, der gegebenenfalls durch Sauerstoff, Schwefel, die NH-Gruppe oder die $NR^6$-Gruppe unterbrochen ist, wobei $R^6$ die oben angegebene Bedeutung besitzt,

sowie ihre pharmazeutisch unbedenklichen Salze.

2) Verbindungen der allgemeinen Formel (I) gemäß Anspruch 1, in welcher

R für Wasserstoff, geradkettiges oder verzweigtes Alkyl mit bis zu 6 Kohlenstoffatomen steht, welches gegebenenfalls 1- oder 2-fach substituiert ist durch Phenyl, Hydroxy, Alkoxy mit 1 bis 4 Kohlenstoffatomen, Amino, Monoalkylamino, Dialkylamino oder Benzylalkylamino, wobei die genannten Alkylgruppen 1 bis 4 Kohlenstoffatome enthalten oder für einen Phenyl- oder Benzylrest steht, wobei der Phenylring gegebenenfalls 1- oder 2-fach substituiert ist durch gleiche oder verschiedene Substituenten aus der Gruppe Fluor, Chlor, Brom, Alkyl, Alkoxy, Alkylmercapto mit jeweils 1 bis 4 Kohlenstoffatomen oder durch Trifluormethyl und

R und $R^1$ gemeinsam gegebenenfalls einen carbocyclischen Ring mit 5 bis 7 Ringgliedern bilden, an den ein Benzol- oder Naphthalinring ankondensiert ist, wobei der Benzol- bzw. Naphthalinring gegebenenfalls 1- oder 2-fach substituiert ist durch gleiche oder verschiedene Substituenten aus der Gruppe Fluor, Chlor, Brom, Trifluormethyl, Alkyl oder Alkoxy mit jeweils 1 bis 4 Kohlenstoffatomen, und der gegebenenfalls durch Sauerstoff, Schwefel, die Gruppe NH oder ein alkylsubstituiertes Stickstoffatom mit 1 bis 4 Kohlenstoffatomen unterbrochen ist,

$R^1$ und $R^2$ gleich oder verschieden sind und jeweils für Wasserstoff, geradkettiges und verzweigtes Alkyl, welcher gegebenenfalls 1 oder 2 gleiche oder verschiedene Substituenten aus der Gruppe Phenyl, Hydroxy, Amino, Carboxy, Halogen, Alkylamino, Dialkylamino mit jeweils 1 bis 4 C-Atomen trägt, oder für Phenyl, Naphthyl, Furyl oder Pyridyl stehen, wobei diese aromatischen Ringe gegebenenfalls 1 oder 2 gleiche oder verschiedene Substituenten aus der Gruppe Nitro, Cyano, Azido, Hydroxy, Amino, Carboxy, Halogen, Phenyl, Alkyl, Alkoxy, Trifluormethyl, Trifluormethoxy, Benzoyloxy, Alkanoyloxy, Trifluormethylthio, Alkylmercapto, Alkylsulfonyl oder Alkylamino substituiert sind, wobei die genannten Alkyl- und Alkoxyreste jeweils 1 bis 4 Kohlenstoffatome enthalten, und ihrerseits substituiert sind durch Hydroxy, Alkoxy, Alkanoyloxy mit jeweils 1 bis 4 C-Atomen, Benzoyloxy oder die Gruppe 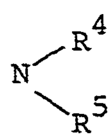, mit den unten angegebenen Bedeutungen für $R^4$ und $R^5$,

m und n gleich oder verschieden sind und jeweils 0 oder 1 bedeuten und

Y für Sauerstoff, Schwefel oder die Gruppe NH steht, und

$R^3$ für geradkettiges oder verzweigtes Alkyl oder Alkenyl mit bis zu 6 Kohlenstoffatomen steht, gegebenenfalls substituiert durch Hydroxy, Alkoxy, (1-4 C-Atome), Fluor, Chlor, Brom, Phenyl, Furyl, Carboxy, Carbalkoxy oder die

Gruppe 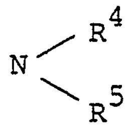 , mit den unten angegebenen Bedeutungen für $R^4$ und $R^5$, oder für Phenyl, Furyl, Pyridyl oder Naphthyl steht, wobei diese aromatischen Ringe gegebenenfalls 1 oder 2 gleiche oder verschiedene Substituenten aus der Gruppe Cyano, Hydroxy, Amino, Carboxy, Halogen, Phenyl, Alkyl, Alkoxy, Trifluormethyl, Trifluormethoxy, Trifluormethylthio, Alkylamino trägt, wobei die genannten Alkyl- und Alkoxyreste jeweils 1 bis 4 Kohlenstoffatome enthalten, steht oder

$R^3$ Wasserstoff bedeutet, wenn

a) R und $R^1$ gemeinsam einen Ring bilden,

b) $R^1$ oder $R^2$ Wasserstoff oder gegebenenfalls substituiertes Alkyl bedeuten, oder

c) $R^1$ und $R^2$ für $R^{1'}$ und $R^{2'}$ stehen, wobei mindestens einer dieser Reste Phenyl bedeutet, welches 1 oder 2 gleiche oder verschiedene Substituenten aus der Gruppe Acyl mit 1 bis 4 Kohle--toffatomen, Hydroxyalkyl, Hydroxyalkoxy mit jeweils 1 bis 4 Kohlenstoffatomen, wobei die Hydroxylgruppen gegebenenfalls durch

Benzoyl oder Alkanoyl mit 1 bis 4 Kohlenstoffatomen substituiert sind, oder die Gruppe $NR^{4'}R^{5'}$, wobei $R^{4'}$ und $R^{5'}$ gemeinsam mit dem Stickstoffatom einen 4- bis 7-gliedrigen Ring bilden, der gegebenenfalls durch Sauerstoff, Schwefel, die NH-Gruppe, die N-Alkyl(1-4 C-Atome)-Gruppe oder die N-Phenylgruppe unterbrochen ist, und

$R^4$ und $R^5$ gleich oder verschieden sind und jeweils für Wasserstoff, Benzyl, Alkyl oder Alkenyl mit bis zu 6 Kohlenstoffatomen stehen, wobei die Alkyl- und Benzylreste gegebenenfalls substituiert sind durch die Gruppe $COR^5$, wobei $R^5$ Alkyl mit 1 bis 4 Kohlenstoffatomen, Benzyl oder Phenyl bedeutet oder

$R^4$ und $R^5$ gemeinsam mit dem Stickstoffatom einen 5- bis 7-gliedrigen Ring bilden, der gegebenenfalls durch Sauerstoff, Schwefel, NH oder $NR^6$ unterbrochen ist, wobei $R^6$ die oben angegebene Bedeutung besitzt.

3) Verbindungen der allgemeinen Formel (I) gemäß Anspruch 1, in welcher

R für Wasserstoff, Alkyl mit 1 bis 4 Kohlenstoffatomen, Benzyl oder Phenyl steht, oder in welcher

R und $R^1$ einen carbocyclischen Ring mit 3 bis 6 Kohlenstoffatomen formen, an welchen ein Benzolring ankondensiert ist,

$R^1$ und $R^2$ gleich oder verschieden sind und jeweils für Wasserstoff, Alkyl mit 1 bis 4 Kohlenstoffatomen, Pyridyl, Furyl oder für Phenyl stehen, wobei der Phenylring gegebenenfalls 1 oder 2 gleiche oder verschiedene Substituenten aus der Gruppe Fluor, Chlor, Nitro, Cyano, Amino, Azido, Trifluormethyl, Trifluormethoxy, Trifluormethylthio, Alkylmercapto und Alkoxy mit 1 bis 4 Kohlenstoffatomen je Alkylrest, N-Pyrrolidino, N-Piperidino, N-Phenylpiperazino, N-Morpholino, Hydroxymethyl, N-Pyrrolidinomethyl, N-Piperidinomethyl, N-Phenylpiperazinomethyl, N-Morpholinomethyl, Benzoyloxymethyl, Alkanoyloxymethyl, wobei die Alkylgruppe 1 bis 6 Kohlenstoffatomen haben kann und ihrerseits substituiert sein kann durch Carboxy oder Carbalkoxy trägt,

m und n 0 oder 1 sein können,

Y für Sauerstoff, Schwefel oder die Gruppe NH steht,

$R^3$ nur dann Wasserstoff sein darf, wenn

a) R und $R^1$ einen Ring formen oder

b) $R^1$ oder $R^2$ für Wasserstoff oder Alkyl stehen, oder wenn

c) $R^1$ und $R^2$ für $R^{1'}$ und $R^{2'}$ stehen, wobei mindestens einer der Substituenten Phenyl bedeutet, welches substituiert ist durch 1 oder 2 gleiche oder verschiedene Substituenten aus der Gruppe Acyloxy mit 2 oder 3 C-Atomen, Hydroxyalkyl mit 1 bis 4 C-Atomen, wobei die Hydroxygruppe gegebenenfalls durch Benzoyl oder Alkanoyl mit 1 bis 6 C-Atomen substituiert ist, wobei der Alkanoylrest seinerseits wieder eine Carboxy oder Carbalkoxy mit 2 bis 4 C-Atomen enthalten kann, N-Pyrrolidonomethyl, N-Piperidinomethyl oder N-Morpholinomethyl,

und im übrigen

$R^3$ für geradkettiges oder verzweigtes Alkyl bzw. Alkenyl mit bis zu 6 Kohlenstoffatomen steht, welches gegebenenfalls substituiert ist durch Hydroxy, Alkoxy, Fluor, Chlor, Brom, Phenyl, Carboxy, Carbalkoxy, N-Pyrrolidono, N-Piperidino, N-Morpholino,
oder für Phenyl steht, welches gegebenenfalls substituiert ist durch Fluor, Chlor, Brom, Hydroxy, Alkoxy und Alkyl mit 1 bis 4 Kohlenstoffatomen, Amino, Alkylamino und Dialkylamino mit je 1 bis 4 Kohlenstoffatomen, Trifluormethyl, Cyano, Nitro oder Carboxy,
und

$R^4$ und $R^5$ gleich oder verschieden sind und für Alkyl mit 1 bis 4 Kohlenstoffatomen oder Benzyl stehen
oder

$R^4$ und $R^5$ gemeinsam mit dem Stickstoffatomen einen 5- oder 6-gliedrigen Ring bilden, der gegebenenfalls durch die Gruppe NH oder $NR^6$ unterbrochen ist, wobei $R^6$ die oben angegebene Bedeutung besitzt.

4) Verfahren zur Herstellung von substituierten 2-Amino-3,4-dihydropyridinderivaten der allgemeinen Formel (I)

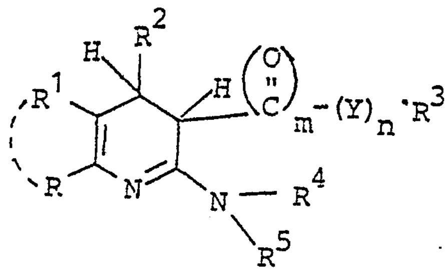

(I)

in welcher

R für Wasserstoff, Alkyl, gegebenenfalls substituiert durch Hydroxy, Halogen, Alkoxy, Aryl oder die Gruppe

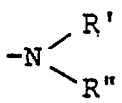 steht,

wobei R' und R" gleich oder verschieden sind und jeweils Wasserstoff, Alkyl oder Aralkyl bedeuten, oder für gegebenenfalls substituiertes Aralkyl steht, oder für Aryl steht, welches gegebenenfalls durch 1 oder 2 gleiche oder verschiedene Substituenten aus der Gruppe Halogen, Alkyl, Alkoxy, Alkylmercapto oder Trifluormethyl substituiert ist, oder

R und $R^1$ gemeinsam gegebenenfalls einen carbocyclischen Ring bilden, der gegebenenfalls substituiert ist durch Hydroxy, Halogen, Nitro, Alkyl, Alkoxy oder die Gruppe 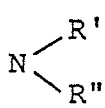, wobei R' und R" die oben angeführten Bedeutungen haben, und wobei an den carbocyclischen Ring ein Arylring ankondensiert sein kann, der gegebenenfalls substituiert ist durch 1 oder 2 gleiche oder verschiedene Substituenten aus der Gruppe Halogen, Alkyl, Alkoxy, Alkylmercapto oder Trifluormethyl, und wobei der Arylring gegebenenfalls durch Sauerstoff, Schwefel oder die $NR^6$-Gruppe unterbrochen ist, wobei $R^6$ die für $R^1$ und $R^2$ angegebene Bedeutung besitzt,

$R^1$ und $R^2$ gleich oder verschieden sind und jeweils für Wasserstoff oder Alkyl, gegebenenfalls substituiert durch Aryl, Heteroaryl, Hydroxyl, Halogen, Alkoxy, Carboxy, Cyano, Carbalkoxy oder die

Gruppe $-N\genfrac{}{}{0pt}{}{R^4}{R^5}$ mit den unten angeführten Bedeutungen für $R^4$ und $R^5$ stehen,

oder

für einen Arylrest oder für einen Heteroarylrest aus der Gruppe Thienyl, Furyl, Pyrryl, Pyrazolyl, Imidazolyl, Oxazolyl, Isooxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl stehen, wobei der Arylrest sowie die Heteroarylreste gegebenenfalls 1, 2 oder 3 gleiche oder verschiedene Substituenten aus der Gruppe

Phenyl, Alkyl, Alkenyl, Alkinyl, Alkoxy, Alkenoxy, Alkinoxy, Alkylen, Dioxyalkylen, Halogen, Acyloxy, Trifluormethylthio, Trifluormethyl, Trifluormethoxy, Hydroxy,

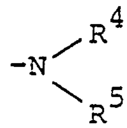 ,

mit den unten angegebenen Bedeutungen für $R^4$ und $R^5$, Nitro, Cyano, Azido, Carboxy, Carbalkoxy, Carbonamido, Sulfonamido oder $SO_{m'}$-Alkyl (m' = 0, 1 oder 2) enthalten, und wobei die Alkyl- und Alkoxysubstituenten ihrerseits gegebenenfalls substituiert sind durch Hydroxy, Alkoxy, Carboxy, Carbalkoxy, Halogen, die

Gruppe 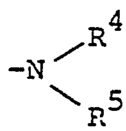 ,

mit den unten angegebenen Bedeutungen für $R^4$ und $R^5$, Aroyloxy oder Alkanoyloxy, wobei der Aroyl- und Alkanoylrest durch Hydroxy, Alkoxy, Halogen, Carboxy oder Carbalkoxy substituiert sein kann,

m und n gleich oder verschieden sind und jeweils 0 oder 1 bedeuten,

Y für Sauerstoff, die Gruppe $SO_m$-$R^6$, (m' = 0, 1, 2) oder die Gruppe N-$R^6$, wobei $R^6$ einen Alkyl- oder Arylrest bedeutet, steht, und



BAD ORIGINAL

$R^3$ für Alkyl, gegebenenfalls substituiert durch Hydroxy, Alkoxy, Halogen, die Gruppe

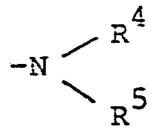 , mit den unten angeführten Bedeutungen

für $R^4$ und $R^5$, Carboxy, Carbalkoxy, Cyano, Alkenyl oder Aryl steht,

oder

für Aryl, welches gegebenenfalls substituiert ist durch 1 oder 2 gleiche oder verschiedene Substituenten der Gruppe Hydroxy, Alkoxy, Halogen, Alkyl, Alkenyl, Aryl, Alkylmercapto, Alkylamino, Carboxy, Carbalkoxy, Cyano, Trifluormethyl, Trifluormethoxy, Trifluormethylthio, steht

oder

$R^3$ auch für Wasserstoff steht, wenn

a) R und $R^1$ gemeinsam einen Ring bilden oder

b) $R^1$ und $R^2$ für Wasserstoff oder gegebenenfalls substituiertes Alkyl stehen, oder

c) $R^1$ und $R^2$ für $R^{1'}$ und $R^{2'}$ stehen, wobei mindestens einer dieser Substituenten für Aryl steht, welches 1, 2 oder 3 gleiche oder verschiedene Substituenten aus der Gruppe Acyloxy, Arylmercapto, Hydroxyalkyl, Hydroxyalkoxy, Hydroxyalkylmercapto und $NR^{4'}R^{5'}$ trägt,

wobei

die Hydroxylgruppen der Alkyl- und Alkoxysubstituenten gegebenenfalls ihrerseits substituiert sind durch Aroyl oder Alkanoyl, die ihrerseits wieder eine Carboxy- oder Carbalkoxygruppe als Substituenten tragen können, und



BAD ORIGINAL

wobei
$R^{4'}$ und $R^{5'}$ mit dem Stickstoffatom einen 4- bis 7-gliedrigen Ring bilden, der gegebenenfalls durch Sauerstoff, Schwefel, die NH-Gruppe oder die $NR^6$-Gruppe unterbrochen ist, wobei $R^6$ die oben angegebene Bedeutung besitzt, und

$R^4$ und $R^5$ gleich oder verschieden sind und jeweils für Wasserstoff oder für einen Alkyl-, Aralkyl-, Alkenyl- oder Alkinylrest stehen, wobei die Alkyl- und Alkenylreste geradkettig, verzweigt oder cyclisch sind und gegebenenfalls substituiert sind durch die Gruppe $COR^5$, wobei $R^5$ Alkyl, Aralkyl oder Aryl bedeutet,

oder

$R^4$ und $R^5$ gemeinsam mit dem Stickstoffatom einen 4- bis 7-gliedrigen Ring bilden, der gegebenenfalls durch Sauerstoff, Schwefel, die NH-Gruppe oder die $NR^6$-Gruppe unterbrochen ist, wobei $R^6$ die oben angegebene Bedeutung besitzt,

sowie ihrer pharmazeutisch unbedenklichen Salze,

dadurch gekennzeichnet, daß man α,ß-ungesättigte Oxoverbindungen der allgemeinen Formel (II)

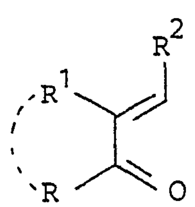

(II)



BAD ORIGINAL

in welcher
R, $R^1$ und $R^2$ die oben angegebene Bedeutung haben,

mit einem 3,3-Diamino-acrylsäureester der allgemeinen Formel (IIIa)

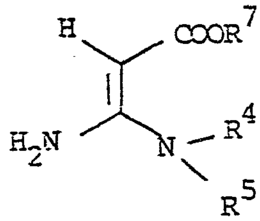

(IIIa)

bzw. ihrer tautomeren Iminform der allgemeinen Formel (IIIb)

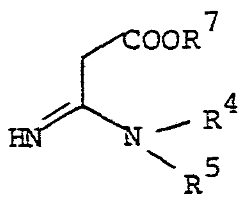

(IIIb)

in welcher
$R^4$ und $R^5$ die oben angegebene Bedeutung haben und
$R^7$ für einen gegebenenfalls substituierten Alkyl- oder Aralkylrest steht,

in Gegenwart von inerten organischen Lösungsmitteln und gegebenenfalls in Gegenwart von sauren oder basischen Katalysatoren bei Temperaturen zwischen 20 und 150°C umsetzt, und die dann erhaltenen Verbindungen der Formel (IV)

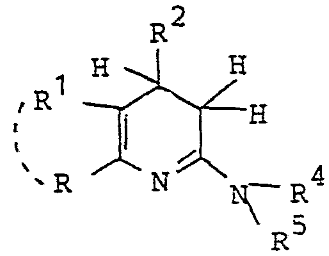

(IV)

in welcher
$R, R^1, R^2, R^4$ und $R^5$ die oben angegebene Bedeutung haben, und für die Herstellung von erfindungsgemäßen Verbindungen, die in 3-Position substituiert sind,

gegebenenfalls mit Verbindungen der allgemeinen Formel (V)

$$X-\left(\overset{O}{\overset{\|}{C}}\right)_m-(Y)_n\cdot R^3 \qquad (V)$$

in welcher
m,n,Y und $R^3$ die oben angegebene Bedeutung haben, und
Y für Halogen, Alkoxy, Aryloxy, Alkylmercapto, Arylmercapto, Toxyl, Mexyl oder die Gruppe $(CO)_m-(Y)_n-R^3$ steht,

in Gegenwart einer starken Base und in Gegenwart eines inerten, aprotischen Lösungsmittels bei Temperaturen zwischen -100°C und 80°C umsetzt.

5) Verfahren gemäß Anspruch 4, dadurch gekennzeichnet, daß man die Reaktionen unter Inertgasatmosphäre in Gegenwart von organischen Lösungsmitteln und sauren oder basischen Katalysatoren durchführt.

6) Verbindungen der allgemeinen Formel (I) gemäß Anspruch 1 zur Verwendung bei der Bekämpfung von Stoffwechselstörungen.

7) Arzneimittel enthaltend mindestens eine Verbindung der allgemeinen Formel (I) gemäß Anspruch 1.

8) Verfahren zur Herstellung von Arzneimitteln, dadurch gekennzeichnet, daß man mindestens eine Verbindung der allgemeinen Formel (I) gemäß Anspruch 1 gegebenenfalls unter Verwendung von Hilfs- und Trägerstoffen in eine geeignete Applikationsform überführt.

9) Verwendung von Verbindungen der allgemeinen Formel (I) gemäß Anspruch 1 bei der Bekämpfung von Erkrankungen.

10) Verwendung von Verbindungen der allgemeinen Formel (I) gemäß Anspruch 1 bei der Bekämpfung von Erkrankungen des Stoffwechsels.

0073997

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung

EP 82107679.1

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A,D | DE - A1 - 2 949 701 (BAYER) (19-06-1981)<br>* Anspruch 1; Seite 14, Zeilen 7-19 *<br>-- | 1,6,7 |
| A,D | DE - A - 2 239 815 (BAYER)<br>* Ansprüche 1,3 *<br>-- | 1,7 |
| A | DE - A1 - 2 514 558 (SANDOZ)<br>* Ansprüche 2,3; Seite 18, Zeilen 18-24 *<br>-- | 1,6,7 |
| A | GB - A - 1 374 339 (SANDOZ)<br>* Seite 1, Zeile 10 - Seite 2, Zeile 1; Seite 3, Zeilen 37-80 *<br>---- | 1,6,7,8 |

KLASSIFIKATION DER ANMELDUNG (Int. Cl.[3])

C 07 D 211/84
C 07 D 221/10
C 07 D 211/90
A 61 K 31/44
C 07 D 215/38

RECHERCHIERTE SACHGEBIETE (Int. Cl.[3])

C 07 D 211/00
C 07 D 221/00
C 07 D 215/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8
Unvollständig recherchierte Patentansprüche:
Nicht recherchierte Patentansprüche:
Grund für die Beschränkung der Recherche: 9,10
(Verfahren zur therapeutischen Behandlung des menschlichen oder tierischen Körpers, Artikel 52(4) EPÜ)

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

| Recherchenort | Abschlußdatum der Recherche | Prüfer |
|---|---|---|
| WIEN | 11-11-1982 | HOCHHAUSER |

EPA Form 1505.1 06.78